(19) Europäisches Patentamt
European Patent Office
Office européen des brevets

(11) Veröffentlichungsnummer: **0 334 138 A1**

# (12) EUROPÄISCHE PATENTANMELDUNG

(21) Anmeldenummer: 89104342.4

(22) Anmeldetag: 11.03.89

(51) Int. Cl.4: C07D 277/68 , C07D 417/12 , A01N 43/78

(30) Priorität: 25.03.88 DE 3810076
20.10.88 DE 3835660

(43) Veröffentlichungstag der Anmeldung: 27.09.89 Patentblatt 89/39

(84) Benannte Vertragsstaaten: BE CH DE FR GB IT LI NL

(71) Anmelder: BAYER AG
D-5090 Leverkusen 1 Bayerwerk(DE)

(72) Erfinder: Wagner, Klaus, Dr.
Jakob-Böhme-Strasse 2
D-5000 Köln 80(DE)
Erfinder: Hänssler, Gerd, Dr.
Am Arenzberg 58a
D-5090 Leverkusen 3(DE)

## (54) 7-Substituierte 2-Carbonamido-benzthiazol-3-oxide.

(57) 7-Substituierte 2-Carbonamido-benzthiazol-3-oxide der allgemeinen Formel (I)

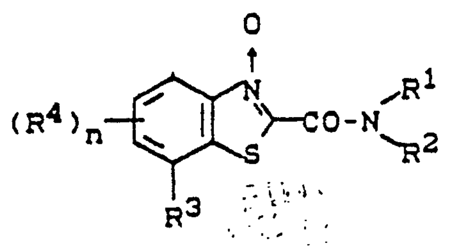

(I)

in welcher
n, $R^1$, $R^2$, $R^3$ und $R^4$ die in der Beschreibung gegebenen Bedeutungen haben, ihre Verwendung in Schädlingsbekämpfungsmitteln und die neuen Zwischenprodukte, die 7-substituierten 2-Alkoxycarbonyl-benzthiazol-3-oxide.

Die neuen Verbindungen sind durch die Formel (I) allgemein definiert und können nach Analogieverfahren hergestellt werden, indem man z. B. geeignete 7-substituierte 2-Alkoxycarbonyl-benzthiazol-3-oxide mit geeigneten Aminen umsetzt. Die ebenfalls neuen 7-substituierten 2-Alkoxycarbonyl-benzthiazol-3-oxide sind durch die Formel (II) allgemein definiert und können z. B. aus geeigneten 2-Chlor-nitrobenzol-Derivaten mit geeigneten Mercaptoessigsäureestern hergestellt werden.

EP 0 334 138 A1

## 7-Substituierte 2-Carbonamido-benzthiazol-3-oxide

Die vorliegende Erfindung betrifft neue 7-substituierte 2-Carbonamido-benzthiazol-3-oxide, ein Verfahren und neue Zwischenprodukte zu ihrer Herstellung sowie ihre Verwendung zur Bekämpfung von Schädlingen.

Bestimmte substituierte Benzthiazol-3-oxide sind bereits als Farbpigmente zum Anfärben von Kunststoffen bekannt geworden (vergl. DE-OS 1 904 653). Eine Anwendung solcher Verbindungen zur Bekämpfung von Schädlingen ist bisher jedoch nicht bekannt.

Es wurden nun neue 7-substituierte 2-Carbonamido-benzthiazol-3-oxide der allgemeinen Formel (I)

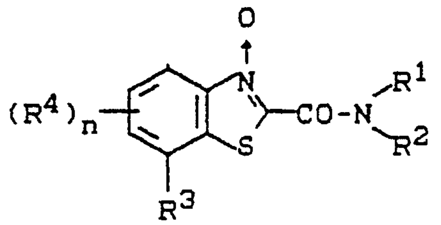

(I)

in welcher

n für die Zahlen 0, 1, 2 oder 3 steht,

$R^1$ für Wasserstoff oder gegebenenfalls substituiertes Alkyl steht,

$R^2$ für Wasserstoff, Hydroxy, Ureido oder für einen gegebenenfalls substituierten Rest aus der Reihe Alkyl, Cycloalkyl, Cycloalkylalkyl, Alkenyl, Alkinyl, Alkoxy, Alkenyloxy, Aralkoxy, Aralkyl, Aryl, Heterocyclyl oder Heterocyclylalkyl steht,

oder

$R^1$ und $R^2$ zusammen für eine gegebenenfalls substituierte und gegebenenfalls durch Heteroatome unterbrochene Alkylenkette stehen,

$R^3$ für Halogen, Alkyl oder Halogenalkyl steht und

$R^4$ für Halogen, Alkyl oder Halogenalkyl steht,

gefunden.

Weiterhin wurde gefunden, daß man die neuen Verbindungen der allgemeinen Formel (I)

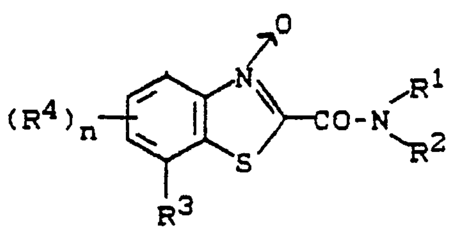

(I)

in welcher

n für die Zahlen 0, 1, 2 oder 3 steht,

$R^1$ für Wasserstoff oder gegebenenfalls substituiertes Alkyl steht,

$R^2$ für Wasserstoff, Hydroxy, Ureido oder für einen gegebenenfalls substituierten Rest aus der Reihe Alkyl, Cycloalkyl, Cycloalkylalkyl, Alkenyl, Alkinyl, Alkoxy, Alkenyloxy, Aralkoxy, Aralkyl, Aryl, Heterocyclyl oder Heterocyclylalkyl steht,

oder

$R^1$ und $R^2$ zusammen für eine gegebenenfalls substituierte und gegebenenfalls durch Heteroatome unterbrochene Alkylenkette stehen,

$R^3$ für Halogen, Alkyl oder Halogenalkyl steht und

$R^4$ für Halogen, Alkyl oder Halogenalkyl steht,

erhält, wenn man 7-substituierte 2-Alkoxycarbonyl-benzthiazol-3-oxide der allgemeinen Formel (II)

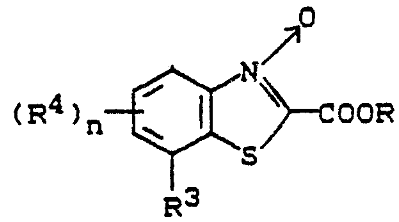

(II)

in welcher
n, $R^3$ und $R^4$ die oben angegebenen Bedeutungen haben und
R für Alkyl steht,
mit Aminen der allgemeinen Formel (III)

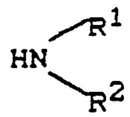

(III)

in welcher
$R^1$ und $R^2$ die oben angegebenen Bedeutungen haben, gegebenenfalls in Gegenwart von Verdünnungsmitteln umsetzt.

Die neuen 7-substituierten 2-Carbonamido-benzthiazol-3-oxide der allgemeinen Formel (I) zeichnen sich durch eine starke biologische Wirkung aus. Überraschenderweise zeigen die erfindungsgemäßen Verbindungen der Formel (I) eine hohe fungizide Wirkung.

Die erfindungsgemäßen 7-substituierten 2-Carbonamidobenzthiazol-3-oxide sind durch die Formel (I) allgemein definiert. Bevorzugt sind Verbindungen der Formel (I), in welcher
n für die Zahlen 0, 1 oder 2 steht,
$R^1$ für Wasserstoff oder für geradkettiges oder verzweigtes Alkyl mit 1 bis 6 Kohlenstoffatomen steht, welches gegebenenfalls ein- bis dreifach, gleich oder verschieden durch Halogen, Hydroxy, $C_1$-$C_4$-Alkoxy, $C_1$-$C_4$-Alkoxy-carbonyl und/oder Di-($C_1$-$C_4$-alkyl)-amino substituiert ist,
$R^2$ für Wasserstoff, Hydroxy, Ureido, für geradkettiges oder verzweigtes Alkyl mit 1 bis 6 Kohlenstoffatomen steht, welches gegebenenfalls ein- bis dreifach, gleich oder verschieden durch Halogen, Hydroxy, Cyano, Benzoyl, $C_1$-$C_4$-Alkoxy, $C_1$-$C_4$-Alkoxy-carbonyl, $C_1$-$C_4$-Alkylamino-carbonyl und/oder Di-($C_1$-$C_4$-alkyl)-amino substituiert ist, für Cycloalkyl mit 3 bis 6 Kohlenstoffatomen oder Cycloalkylalkyl mit 3 bis 6 Kohlenstoffatomen im Cycloalkylteil und 1 bis 3 Kohlenstoffatomen im Alkylteil steht, welche gegebenenfalls ein- bis dreifach, gleich oder verschieden durch Halogen und/ oder $C_1$-$C_4$-Alkyl substituiert sind, für geradkettiges oder verzweigtes Alkenyl mit 3 bis 6 Kohlenstoffatomen steht, welches gegebenenfalls ein- bis dreifach, gleich oder verschieden durch Halogen, Cyano und/oder $C_1$-$C_4$-Alkoxy-carbonyl substituiert ist, für geradkettiges oder verzweigtes Alkinyl mit 3 bis 6 Kohlenstoffatomen steht, welches gegebenenfalls ein- bis dreifach, gleich oder verschieden durch Halogen, Cyano und/oder $C_1$-$C_4$-Alkoxy-carbonyl substituiert ist, für geradkettiges oder verzweigtes Alkoxy mit 1 bis 6 Kohlenstoffatomen steht, welches gegebenenfalls ein- bis dreifach, gleich oder verschieden durch Halogen, Cyano, $C_1$- $C_4$-Alkoxy und/oder $C_1$-$C_4$-Alkoxy-carbonyl substituiert ist, für geradkettiges oder verzweigtes Alkenyloxy mit 3 bis 6 Kohlenstoffatomen steht, welches gegebenenfalls ein- bis dreifach, gleich oder verschieden durch Halogen substituiert ist, für Benzyloxy steht, welches gegebenenfalls ein- bis dreifach, gleich oder verschieden durch Halogen, Nitro, Cyano, $C_1$-$C_4$-Alkyl, $C_1$-$C_4$-Alkoxy, $C_1$-$C_4$-Alkoxy-carbonyl und/oder Halogenalkyl mit 1 oder 2 Kohlenstoffatomen und 1 bis 5 gleichen oder verschiedenen Halogenatomen substituiert ist, für Aralkyl mit 6 bis 10 Kohlenstoffatomen im Arylteil und 1 bis 4 Kohlenstoffatomen im geradkettigen oder verzweigten Alkylteil steht, welches gegebenenfalls im Aryl-und/oder Alkylteil jeweils ein- bis dreifach, gleich oder verschieden durch Halogen, Hydroxy, Phenyl, Nitro, Cyano, $C_1$-$C_4$-Alkyl, $C_1$-$C_4$-Alkoxy, $C_1$-$C_4$-Alkoxy-carbonyl und/oder Halogenalkyl mit 1 oder 2 Kohlenstoffatomen und 1 bis 5 gleichen oder verschiedenen Halogenatomen substituiert ist, für Phenyl oder Naphthyl steht, welche gegebenenfalls ein- bis dreifach, gleich oder verschieden durch Halogen, Hydroxy, Nitro, Cyano, $C_1$-$C_4$-Alkyl, Halogenalkyl mit 1 oder 2 Kohlenstoffatomen und 1 bis 5 gleichen oder verschiedenen Halogenatomen, $C_1$-$C_4$-Alkoxy, $C_1$-$C_2$-Halogenalkoxy, $C_1$-$C_4$-Alkylthio, $C_1$-$C_2$-Halogenalkylthio und/oder $C_1$-$C_4$-Alkoxy-carbonyl substituiert sind, oder für einen gegebenenfalls durch Halogen, Nitro, Cyano, $C_1$-$C_4$-Alkyl, $C_1$-$C_4$-Alkoxy und/oder $C_1$-$C_4$-Alkoxy-carbonyl ein-bis dreifach, gleich

oder verschieden substituierten Ein- oder Zweiring-Heterocyclus steht, wie Furyl, Thienyl, Oxazolyl, Thiazolyl, Oxadiazolyl, Thiadiazolyl, Pyridyl, Pyrimidinyl, Pyrrolidinyl, Thiomorpholinyl, Morpholinyl

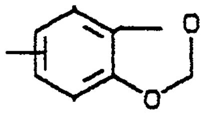 oder 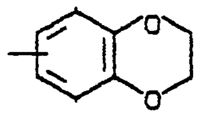

welche gegebenenfalls über eine geradkettige oder verzweigte Alkylenkette mit 1 bis 4 Kohlenstoffatomen gebunden sind
oder
$R^1$ und $R^2$ zusammen für eine gegebenenfalls ein- bis dreifach, gleich oder verschieden durch $C_1$-$C_4$-Alkyl substituierte und gegebenenfalls durch ein oder mehrere Heteroatome, wie z.B. Sauerstoff, unterbrochene Alkylenkette mit 2 bis 8 Ringgliedern stehen,
$R^3$ für Halogen, Methyl, Ethyl oder Halogenalkyl mit 1 bis 3 Kohlenstoffatomen und 1 bis 6 gleichen oder verschiedenen Halogenatomen steht und
$R^4$ für Halogen, Methyl, Ethyl oder für Halogenalkyl mit 1 bis 3 Kohlenstoffatomen und 1 bis 6 gleichen oder verschiedenen Halogenatomen steht.

Besonders bevorzugt sind Verbindungen der Formel (I),
in welcher
n für die Zahlen 0, 1 oder 2 steht,
$R^1$ für Wasserstoff oder für geradkettiges oder verzweigtes Alkyl mit 1 bis 4 Kohlenstoffatomen steht, welches gegebenenfalls ein- oder zweifach, gleich oder verschieden durch Hydroxy, $C_1$-$C_2$-Alkoxy oder $C_1$-$C_2$-Alkoxy-carbonyl substituiert ist,
$R^2$ für Wasserstoff, Hydroxy, Ureido, für geradkettiges oder verzweigtes Alkyl mit 1 bis 6 Kohlenstoffatomen steht, welches gegebenenfalls ein- oder zweifach, gleich oder verschieden durch Hydroxy, Cyano, Benzoyl, $C_1$-$C_2$-Alkoxy, $C_1$-$C_2$-Alkoxy-carbonyl, $C_1$-$C_2$-Alkylamino-carbonyl oder Di-($C_1$-$C_2$-alkyl)-amino substituiert ist, für Cycloalkyl mit 3 bis 6 Kohlenstoffatomen, für Cycloalkylmethyl oder -ethyl mit 3 bis 6 Kohlenstoffatomen im Cycloalkylteil, für geradkettiges oder verzweigtes Alkenyl mit 3 bis 6 Kohlenstoffatomen, für geradkettiges oder verzweigtes Alkinyl mit 3 bis 6 Kohlenstoffatomen, für geradkettiges oder verzweigtes Alkoxy mit 1 bis 6 Kohlenstoffatomen, für geradkettiges oder verzweigtes Alkenyloxy mit 3 bis 6 Kohlenstoffatomen oder für Benzyloxy steht, welches gegebenenfalls ein- oder zweifach, gleich oder verschieden durch Fluor, Chlor, Methyl und/oder Methoxy substituiert ist, für Phenylalkyl mit 1 bis 3 Kohlenstoffatomen im Alkylteil steht, welches gegebenenfalls ein- bis dreifach, gleich oder verschieden im Phenyl- und/oder Alkylteil durch Fluor, Chlor, Hydroxy, Phenyl, $C_1$-$C_4$-Alkyl, $C_1$-$C_2$-Alkoxy, Trifluormethyl und/oder $C_1$-$C_2$-Alkoxy-carbonyl substituiert ist, für Naphthylmethyl oder für Phenyl steht, welches gegebenenfalls ein- bis dreifach, gleich oder verschieden durch Fluor, Chlor, Brom, Hydroxy, $C_1$-$C_4$-Alkyl, Trifluormethyl, $C_1$-$C_2$-Alkoxy, Trifluormethoxy, $C_1$-$C_2$-Alkylthio und/oder Trifluormethylthio substituiert ist, für Furylmethyl, für Pyridylmethyl, für gegebenenfalls ein- oder zweifach durch Methyl substituiertes Morpholinyl, für Morpholinyl-$C_1$-$C_3$-alkyl oder für gegebenenfalls ein- oder zweifach, gleich oder verschieden durch $C_1$-$C_3$-Alkyl substituiertes Pyrrolidinyl-$C_1$-$C_3$-alkyl steht,
für

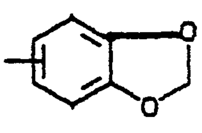 , 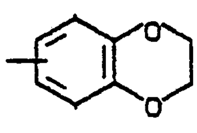 oder 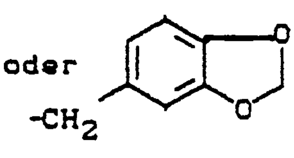

steht
oder
$R^1$ und $R^2$ zusammen mit dem Stickstoffatom, an das sie gebunden sind, für gegebenenfalls ein- bis dreifach, gleich oder verschieden durch Methyl und/oder Ethyl substituiertes Pyrrolidinyl, Piperidinyl oder Morpholinyl stehen,
$R^3$ für Chlor oder Trifluormethyl steht und
$R^4$ für Chlor oder Trifluormethyl steht.

Halogen steht für Fluor, Chlor, Brom oder Iod, bevorzugt für Fluor, Chlor oder Brom, besonders

bevorzugt für Fluor oder Chlor allein und/oder in Zusammensetzungen wie Halogenalkyl, wenn nicht anders definiert. Alle Reste, die substituiert sein können, sind ein- bis mehrfach, gleich oder verschieden, bevorzugt ein- bis dreifach, gleich oder verschieden und besonders bevorzugt ein- oder zweifach, gleich oder verschieden substituiert, wenn nicht anders angegeben.

Beispiele für die erfindungsgemäßen Verbindungen der Formel (I) sind in der nachstehenden Tabelle 1 aufgeführt - vergl. auch Herstellungsbeispiele.

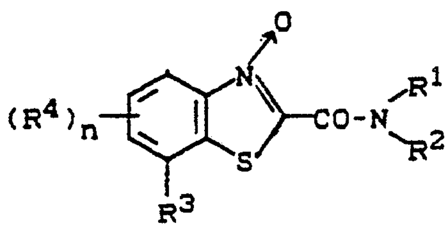

(I)

Tabelle 1: Beispiele für die Verbindungen der Formel (I)

| $R^1$ | $R^2$ | $R^3$ | $R^4$ | n |
|---|---|---|---|---|
| H | H | Cl | - | 0 |
| H | H | Cl | (6-)Cl | 1 |
| H | H | Cl | (5-)Cl | 1 |
| H | H | $CF_3$ | - | 0 |
| H | H | $CF_3$ | (5-)$CF_3$ | 1 |
| H | $CH_3$ | Cl | - | 0 |
| $CH_3$ | $CH_3$ | Cl | - | 0 |
| H | $C_2H_5$ | Cl | - | 0 |
| $C_2H_5$ | $C_2H_5$ | Cl | - | 0 |
| H | $C_3H_7$ | Cl | - | 0 |
| H | $CH(CH_3)_2$ | Cl | - | 0 |
| H | $C_4H_9$ | Cl | - | 0 |
| H | $CH_2CH(CH_3)_2$ | Cl | - | 0 |
| H | $CH(C_2H_5)_2$ | Cl | - | 0 |

Tabelle 1: (Fortsetzung)

| $R^1$ | $R^2$ | $R^3$ | $R^4$ | n |
|---|---|---|---|---|
| H | OH | Cl | - | 0 |
| H | $-NH-CO-NH_2$ | Cl | - | 0 |
| H | 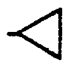 | Cl | - | 0 |
| H | 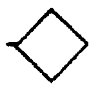 | Cl | - | 0 |
| H | 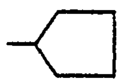 | Cl | - | 0 |
| H | 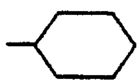 | Cl | - | 0 |
| H | $-CH_2$ 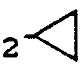 | Cl | - | 0 |
| H | $-CH_2$ 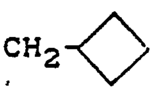 | Cl | - | 0 |
| H | $-CH_2$ 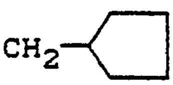 | Cl | - | 0 |
| H | $-CH_2$ 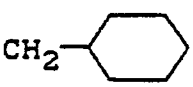 | Cl | - | 0 |
| H | $-CH_2-CH=CH_2$ | Cl | - | 0 |
| H | $-CH_2-C\equiv CH$ | Cl | - | 0 |
| H | $OCH_3$ | Cl | - | 0 |
| H | $OC_2H_5$ | Cl | - | 0 |

<u>Tabelle 1:</u> (Fortsetzung)

| $R^1$ | $R^2$ | $R^3$ | $R^4$ | n |
|---|---|---|---|---|
| H | $OC_3H_7$-n | Cl | - | 0 |
| H | $OCH(CH_3)_2$ | Cl | - | 0 |
| H | $OC_4H_9$-n | Cl | - | 0 |
| H | $OCH_2$-$CH(CH_3)_2$ | Cl | - | 0 |
| H | $OCH_2$-$CH$=$CH_2$ | Cl | - | 0 |
| H | $OCH_2$ 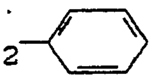 | Cl | - | 0 |
| H | $-CH_2$ 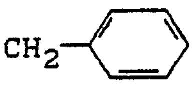 | Cl | - | 0 |
| $CH_3$ | $-CH_2$ 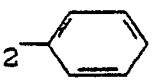 | Cl | - | 0 |
| H | 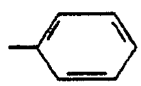 | Cl | - | 0 |
| H | 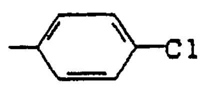 | Cl | - | 0 |
| H | $-CH_2$ 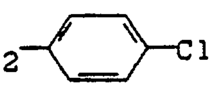 | Cl | - | 0 |
| H | 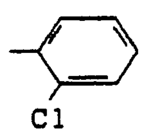 | Cl | - | 0 |

Tabelle 1: (Fortsetzung)

| $R^1$ | $R^2$ | $R^3$ | $R^4$ | n |
|---|---|---|---|---|
| H | 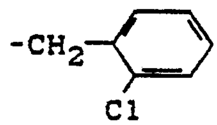 | Cl | - | 0 |
| $-(CH_2)_4-$ | | Cl | - | 0 |
| $-(CH_2)_5-$ | | Cl | - | 0 |
| H | 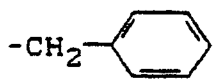 | Cl | (6-)Cl | 1 |
| H | 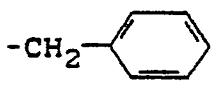 | $CF_3$ | (5-)$CF_3$ | 1 |
| H | 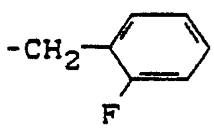 | Cl | - | 0 |
| H | 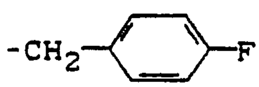 | Cl | - | 0 |
| H | 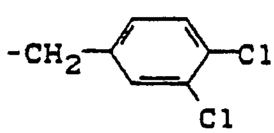 | Cl | - | 0 |
| H | 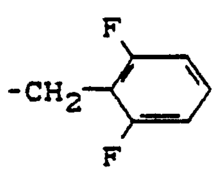 | Cl | - | 0 |

Tabelle 1: (Fortsetzung)

| $R^1$ | $R^2$ | $R^3$ | $R^4$ | n |
|---|---|---|---|---|
| H | 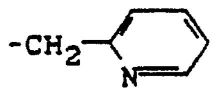 | Cl | - | 0 |
| H | 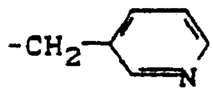 | Cl | - | 0 |
| H | 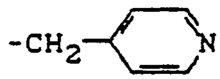 | Cl | - | 0 |
| H | 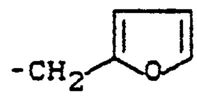 | Cl | - | 0 |
| H | 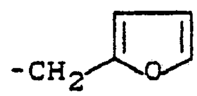 | $CF_3$ | - | 0 |
| H | $-CH_2-COOCH_3$ | Cl | - | 0 |
| H | $-CH_2-COOC_2H_5$ | Cl | - | 0 |
| H | $-CH-COOCH_3$<br>$\vert$<br>$CH_3$ | Cl | - | 0 |
| H | $-CH-COOC_2H_5$<br>$\vert$<br>$CH_3$ | Cl | - | 0 |
| H | 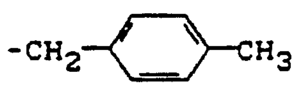 | Cl | - | 0 |
| H | 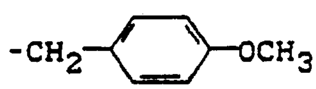 | Cl | - | 0 |

Tabelle 1: (Fortsetzung)

| $R^1$ | $R^2$ | $R^3$ | $R^4$ | n |
|---|---|---|---|---|
| H | $-CH_2-CH_2-OH$ | Cl | - | 0 |
| $-CH_2-CH_2-OH$ | $-CH_2-CH_2-OH$ | Cl | - | 0 |
| H | $-CH_2-CH_2-OCH_3$ | Cl | - | 0 |
| $-CH_2-CH_2-OCH_3$ | $-CH_2CH_2OCH_3$ | Cl | - | 0 |
| H | 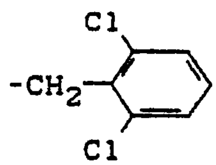 | Cl | - | 0 |
| H | $-CH_2-CH_2-N(C_2H_5)_2$ | Cl | - | 0 |
| H | 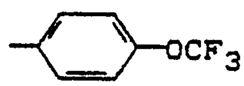 | Cl | - | 0 |
| H | 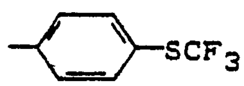 | Cl | - | 0 |
| H | 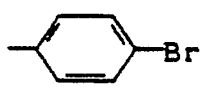 | Cl | - | 0 |
| H | 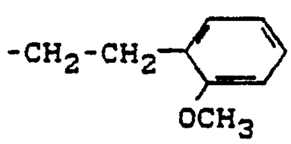 | Cl | - | 0 |
| H | 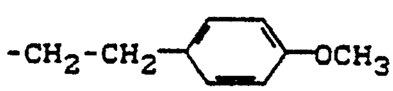 | Cl | - | 0 |
| H | 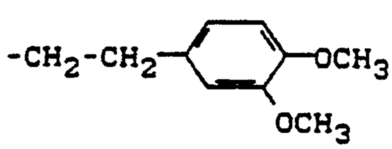 | Cl | - | 0 |

Tabelle 1: (Fortsetzung)

| $R^1$ | $R^2$ | $R^3$ | $R^4$ | n |
|---|---|---|---|---|
| H | 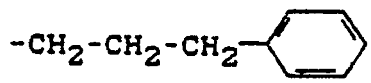 | Cl | - | 0 |
| H | $-CH_2CH_2CH(C_6H_5)_2$ | Cl | - | 0 |
| H | 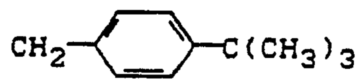 | Cl | - | 0 |
| H | 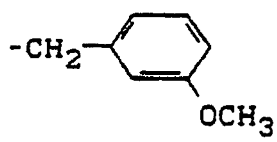 | Cl | - | 0 |
| H | 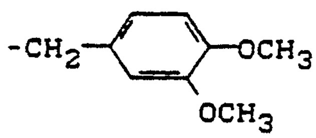 | Cl | - | 0 |
| H | 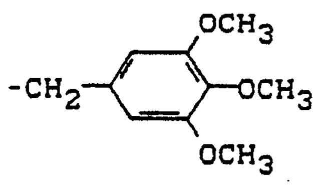 | Cl | - | 0 |
| H | 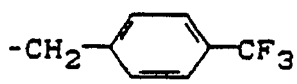 | Cl | - | 0 |
| H | 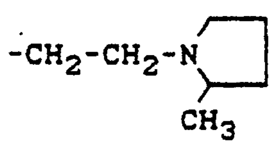 | Cl | - | 0 |

Verwendet man beispielsweise 7-Chlor-2-methoxycarbonyl-benzthiazol-3-oxid und N,O-Dimethyl-hydroxylamin als Ausgangsstoffe, so kann der Reaktionsverlauf beim erfindungsgemäßen Herstellungsverfahren durch das folgende Formelschema wiedergegeben werden:

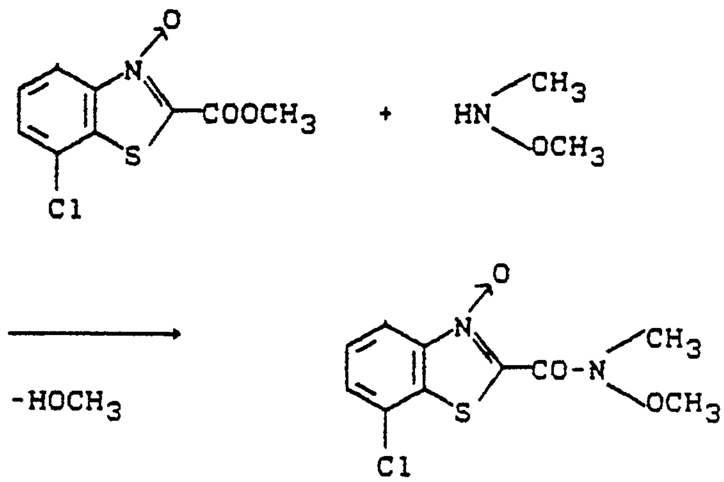


Die beim erfindungsgemäßen Verfahren zur Herstellung von Verbindungen der Formel (I) als Ausgangsstoffe zu verwendenden 7-substituierten 2-Alkoxycarbonyl-benzthiazol-3-oxide sind durch die Formel (II) allgemein definiert.

In der Formel (II) haben n, $R^3$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, $R^3$ und $R^4$ angegeben wurden und R steht vorzugsweise für $C_1$-$C_4$-Alkyl, insbesondere für Methyl.

Als Beispiele für die Ausgangsstoffe der Formel (II) seien genannt:

7-Chlor-, 5,7-Dichlor-, 6,7-Dichlor-, 4,6,7-Trichlor-, 7-Trifluormethyl- und 5,7-Bis-trifluormethyl-2-methoxycarbonyl-, -2-ethoxycarbonyl-, -2-propoxycarbonyl- und -2-butoxycarbonyl-benzthiazol-3-oxid.

Die Ausgangsstoffe der Formel (II) sind noch nicht aus der Literatur bekannt. Man erhält die neuen 7-substituierten 2-Alkoxycarbonyl-benzthiazol-3-oxide der allgemeinen Formel (II), wenn man 2-Chlor-nitrobenzol-Derivate der allgemeinen Formel (IV)

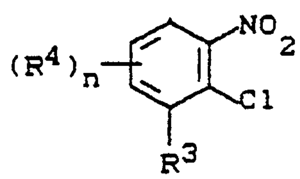 (IV)

in welcher

n, $R^3$ und $R^4$ die oben angegebenen Bedeutungen haben,

mit Mercaptoessigsäureestern der allgemeinen Formel (V)

$HS$-$CH_2$-$COOR$ (V)

in welcher

R die oben angegebene Bedeutung hat,

in Gegenwart einer Base, wie z. B. Triethylamin, und in Gegenwart eines Verdünnungsmittels, wie z. B. Dimethylsulfoxid, Benzol, Toluol, Tetrahydrofuran, Dioxan, Methanol, Ethanol, Propanol, Isopropanol und/oder Wasser, bei Temperaturen zwischen 0 °C und 100 °C, vorzugsweise zwischen 20 °C und 80 °C, umsetzt.

In der Formel (IV) haben n, $R^3$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, $R^3$ und $R^4$ angegeben wurden.

Als Beispiele für die Verbindungen der Formel (IV) seien genannt:

2,3-Dichlor-, 2,3,4-Trichlor-, 2,3,5-Trichlor-, 2,3,5,6-Tetrachlor-, 2-Chlor-3-trifluormethyl- und 2-Chlor-3,5-bis-trifluormethyl-nitrobenzol.

Die 2-Chlor-nitrobenzol-Derivate der Formel (IV) sind bekannte organische Synthesechemikalien.

In Formel (V) steht R vorzugsweise für $C_1$-$C_4$-Alkyl, insbesondere für Methyl.

Als Beispiele für die Verbindungen der Formel (V) seien genannt:

Mercaptoessigsäure-methylester, -ethylester, -propylester und -butylester.

Die Mercaptoessigsäureester der Formel (V) sind bekannte organische Synthesechemikalien.

Die beim erfindungsgemäßen Verfahren zur Herstellung von Verbindungen der Formel (I) weiter als Ausgangsstoffe zu verwendenden Amine sind durch die Formel (III) allgemein definiert.

In der Formel (III) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Als Beispiele für die Ausgangsstoffe der Formel (III) seien genannt:

Methylamin, Ethylamin, Propylamin, Isopropylamin, Butylamin, Isobutylamin, sec-Butylamin, tert-Butylamin, Pentylamin, Isopentylamin, sec-Pentylamin, tert-Pentylamin, 1-Ethyl-propylamin, Hexylamin, Isohexylamin, Dimethylamin, Diethylamin, Dipropylamin, Diisopropylamin, Dibutylamin, Diisobutylamin, N-Methyl-ethylamin, N-Methyl propylamin, N-Methyl-butylamin, N-Methyl-isopropylamin, N-Methyl-isobutylamin, N-Methyl-sec-butylamin, Benzylamin, N-Methyl-benzylamin, Hydroxylamin, O-Methyl-, O-Ethyl-, O-Propyl-, O-Isopropyl-, O-Butyl-, O-Isobutyl-, O-sec-Butyl-, O-Pentyl-, O-Hexyl-, O-Allyl-, O-Benzyl-, O-(4-Fluor-benzyl)-, O-(4-Chlor-benzyl)- und O-(4-Methyl-benzyl)-hydroxylamin, N,O-Dimethyl-hydroxylamin, Anilin, 4-Fluor- und 2-Fluor-anilin, 2-Chlor-, 3-Chlor-, 4-Chlor-, 3,4-Dichlor-, 2,4-Dichlor-, 2,6-Dichlor-, 2,4,5-Trichlor- und 3,5-Dichlor-anilin, 4-Brom-anilin, 2-Methyl-, 3-Methyl- und 4-Methyl-anilin, 2-Trifluormethyl-, 3-Trifluormethyl- und 4-Trifluormethyl-anilin, 2-Methoxy-, 3-Methoxy- und 4-Methoxy-anilin, 3-Trifluormethoxy-, und 4-Trifluormethoxy-anilin, 4-Methylthio- und 4-Trifluormethylthio-anilin, 2-Chlor-, 3-Chlor- und 4-Chlor-benzylamin, Semicarbazid, Morpholin, Pyrrolidin, Piperidin, 2-Fluor- und 4-Fluor-benzylamin, 2-Aminomethyl-, 3-Aminomethyl- und 4-Aminomethyl-pyridin, 2-Aminomethyl-furan, 2,2-Dimethyl-propylamin, Cyclopropylamin, Cyclobutylamin, Cyclopentylamin, Cyclohexylamin, Cyclopropyl-methylamin, Cyclobutyl-methylamin, Cyclopentyl-methylamin, Cyclohexyl-methylamin, Aminoessigsäure-methylester und -ethylester, 1-Amino-propionsäure-methylester und -ethylester, 2-Methyl- und 4-Methyl-benzylamin, 2-Methoxy-, 3-Methoxy-und 4-Methoxy-benzylamin, 1-Phenyl- und 2-Phenyl-ethylamin, 1-Aminomethyl-naphthalin, 2-Hydroxy-2-phenyl-ethylamin, 3,4-Methylendioxy-benzylamin, 4-tert-Butyl-benzylamin, N-Amino-morpholin, N-(3-Aminopropyl)-morpholin, 3,4-Dimethoxy- und 3,4,5-Trimethoxy-benzylamin, 2-Hydroxy-propylamin, 4-Trifluormethyl-benzylamin, 3,5-Dimethyl-benzylamin, 2-(3-Methoxy-phenyl)-ethylamin, 2-(3-Chlor-phenyl)-ethylamin, 2-(3-Trifluormethyl-phenyl)-ethylamin, 2-Hydroxy-ethylamin, 2,6-Dichlor-4-trifluormethyl-benzylamin, 2-(2-Fluor-phenyl)-ethylamin, 2-(2,4-Difluor-phenyl)-ethylamin, 2-(2-Trifluormethyl-phenyl)-ethylamin, 2-(4-Trifluormethyl-phenyl)-ethylamin, 2-(3-Fluor-phenyl)-ethylamin, N,N-Dimethyl- und N,N-Diethylethylendiamin, 2-(3,4-Dimethoxy-phenyl)-ethylamin, 3,5-Dimethyl-morpholin, 1-Ethyl-2-aminomethyl-pyrrolidin und 3-Phenyl-propylamin.

Die Amine der Formel (III) sind bekannte organische Synthesechemikalien.

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei neben Wasser praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon, Alkohole wie Methanol, Ethanol, Propanol, Isopropanol, Butanol, Isobutanol, sec-Butanol und tert-Butanol sowie Dimethylsulfoxid, Tetramethylensulfon und Hexamethylphosphorsäuretriamid.

Die Reaktionstemperaturen können bei dem erfindungsgemäßen Verfahren zur Herstellung der Verbindungen der Formel (I) in einem größeren Bereich variiert werden. Im allgemeinen arbeitet man bei Temperaturen zwischen 0 °C und 100 °C, vorzugsweise bei Temperaturen zwischen 20 °C und 80 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die erfindungsgemäßen Wirkstoffe weisen eine starke biologische Wirkung auf und können zur Bekämpfung von unerwünschten Schädlingen praktisch eingesetzt werden. Die Wirkstoffe sind u. a. für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Verbindungen der Formel (I) zeigen insbesondere starke protektive Wirkung gegen Pyricularia-Arten wie z. B. Pyricularia oryzae, welche im Reisanbau Schäden verursachen. Auch gegen Venturia- und Phytophthora-Arten wird eine gute Wirkung beobachtet.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem

Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalo cyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 96 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

## Herstellungsbeispiele

### Beispiel 1

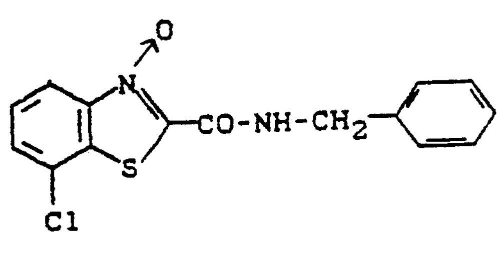

3,0 g (0,028 Mol) Benzylamin werden unter Rühren zu einer Mischung aus 6,1 g (0,025 Mol) 7-Chlor-2-methoxy-carbonyl-benzthiazol-3-oxid und 100 ml Methanol tropfenweise gegeben und das Reaktionsgemisch wird zwei Stunden bei 20 °C gerührt. Das hierbei angefallene kristalline Produkt wird durch Absaugen isoliert.

Man erhält 7,5 g (94 % der Theorie) 7-Chlor-2-benzyl-aminocarbonyl-benzthiazol-3-oxid vom Schmelzpunkt 126 °C.

Analog Beispiel 1 können die in der nachstehenden Tabelle 2 aufgeführten Verbindungen der Formel (I) hergestellt werden.

Tabelle 2: Herstellungsbeispiele für die Verbindungen der Formel (I)

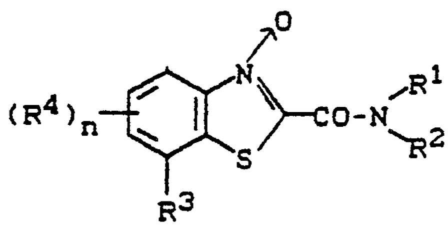

(I)

| Bsp. Nr. | $R^1$ | $R^2$ | $R^3$ | $R^4$ | n | Physik. Daten (Schmelzpunkt (°C) |
|---|---|---|---|---|---|---|
| 2 | H | $CH_3$ | Cl | - | 0 | 205 |
| 3 | H | $-CH(C_2H_5)_2$ | Cl | - | 0 | Öl |
| 4 | H | $C_3H_7$-n | Cl | - | 0 | 146 |
| 5 | H | $-CH(CH_3)_2$ | Cl | - | 0 | 112 |
| 6 | $CH_3$ | $-CH_2-$ 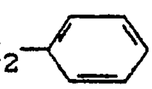 | Cl | - | 0 | Öl |
| 7 | H | OH | Cl | - | 0 | 201 |
| 8 | H | $OCH_3$ | Cl | - | 0 | 179 |
| 9 | H | $-CH(CH_3)CH_2CH_3$ | Cl | - | 0 | Öl |
| 10 | H | $-CH_2-CH(CH_3)_2$ | Cl | - | 0 | 113 |
| 11 | H | 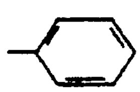 | Cl | - | 0 | 203 |

Tabelle 2: (Fortsetzung)

| Bsp. Nr. | $R^1$ | $R^2$ | $R^3$ | $R^4$ | n | Physik. Daten (Schmelzpunkt - (°C) |
|---|---|---|---|---|---|---|
| 12 | H | $-CH_2-$ 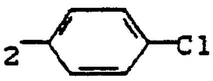 | Cl | - | 0 | 162 |
| 13 | H | $-CH_2-$ 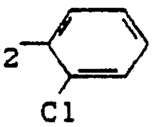 | Cl | - | 0 | 183 |
| 14 | H | $-NHCONH_2$ | Cl | - | 0 | 269 |
| 15 | $-CH_2CH_2-O-CH_2CH_2-$ | | Cl | - | 0 | 178 |
| 16 | H | $-CH_2-$ 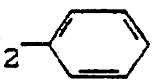 | Cl | (6-)Cl | 1 | 164 |
| 17 | H | $-CH_2-$ 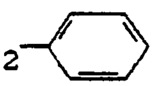 | $CF_3$ | (5-)$CF_3$ | 1 | 123 |
| 18 | H | $-CH_2-$ 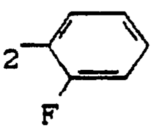 | Cl | - | 0 | 147 |
| 19 | H | $-CH_2-$ 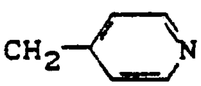 | Cl | - | 0 | 163 |
| 20 | H | $-CH_2-$ 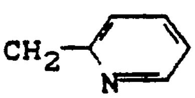 | Cl | - | 0 | 147 |
| 21 | H | $-CH_2-$ 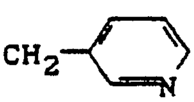 | Cl | - | 0 | 176 |

Tabelle 2: (Fortsetzung)

| Bsp. Nr. | $R^1$ | $R^2$ | $R^3$ | $R^4$ | n | Physik. Daten (Schmelzpunkt (° C) |
|---|---|---|---|---|---|---|
| 22 | H | 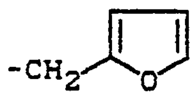 | Cl | - | 0 | 122 |
| 23 | H | $-CH_2C(CH_3)_3$ | Cl | - | 0 | 133 |
| 24 | H | 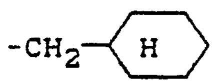 | Cl | - | 0 | 120 |
| 25 | H | 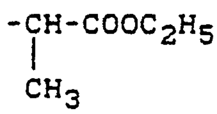 | Cl | - | 0 | 127 |
| 26 | H | 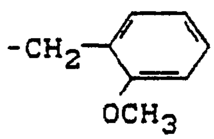 | Cl | - | 0 | 173 |
| 27 | H | 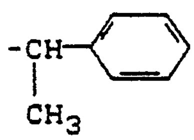 | Cl | - | 0 | 161 |
| 28 | H | 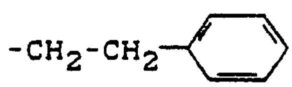 | Cl | - | 0 | 115 |
| 29 | H | 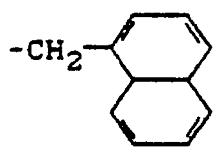 | Cl | - | 0 | 203 |
| 30 | H | 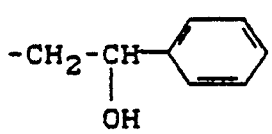 | Cl | - | 0 | 139 |

Tabelle 2: (Fortsetzung)

| Bsp. Nr. | $R^1$ | $R^2$ | $R^3$ | $R^4$ | n | Physik. Daten (Schmelzpunkt (°C) |
|---|---|---|---|---|---|---|
| 31 | H | 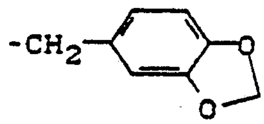 | Cl | - | 0 | 166 |
| 32 | H | 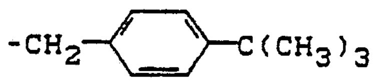 | Cl | - | 0 | 147 |
| 33 | H | 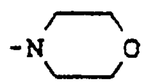 | Cl | - | 0 | 206 |
| 34 | H | 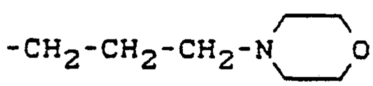 | Cl | - | 0 | 91 |
| 35 | H | 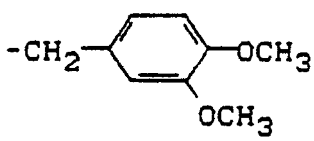 | Cl | - | 0 | 161 |
| 36 | H | 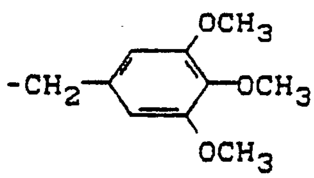 | Cl | - | 0 | 188 |
| 37 | H | $-CH_2-CH-CH_3$<br>OH | Cl | - | 0 | 142 |
| 38 | H | 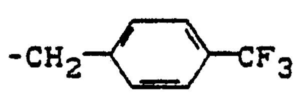 | Cl | - | 0 | 115 |

T a b e l l e 2: (Fortsetzung)

| Bsp. Nr. | $R^1$ | $R^2$ | $R^3$ | $R^4$ | n | Physik. Daten (Schmelzpunkt (° C) |
|---|---|---|---|---|---|---|
| 39 | H | 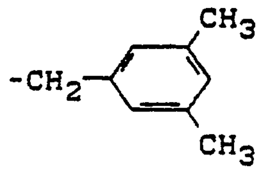 | Cl | - | 0 | 186 |
| 40 | H | 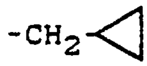 | Cl | - | 0 | 139 |
| 41 | H | 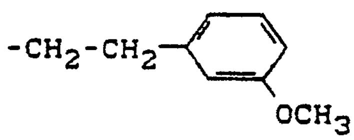 | Cl | - | 0 | 104 |
| 42 | H | 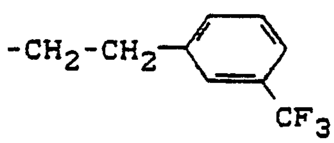 | Cl | - | 0 | 117 |
| 43 | H | 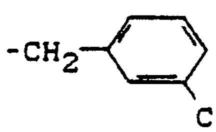 | Cl | - | 0 | 146 |
| 44 | $CH_3$ | $CH_3$ | Cl | - | 0 | 172 |
| 45 | H | $-CH_2-CH_2-OH$ | Cl | - | 0 | 189 |
| 46 | H | 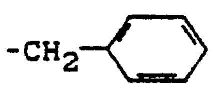 | $CF_3$ | - | 0 | 138 |
| 47 | H | 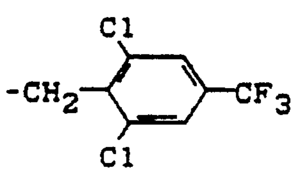 | Cl | - | 0 | 197 |

Tabelle 2: (Fortsetzung)

| Bsp. Nr. | $R^1$ | $R^2$ | $R^3$ | $R^4$ | n | Physik. Daten (Schmelzpunkt (°C) |
|---|---|---|---|---|---|---|
| 48 | H | 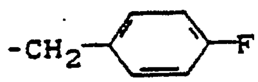 | Cl | - | 0 | 151 |
| 49 | H | 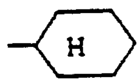 | Cl | - | 0 | 108 |
| 50 | H | 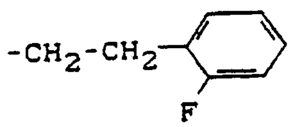 | Cl | - | 0 | 117 |
| 51 | H | 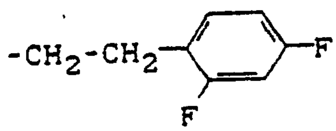 | Cl | - | 0 | 125 |
| 52 | H | 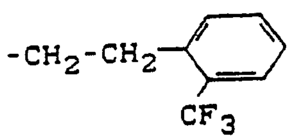 | Cl | - | 0 | 160 |
| 53 | H | 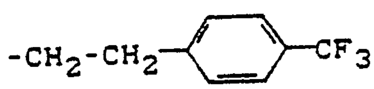 | Cl | - | 0 | 146 |
| 54 | H | 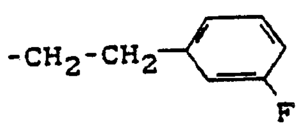 | Cl | - | 0 | 129 |
| 55 | H | $-CH_2-CH_2-N(CH_3)_2$ | Cl | - | 0 | 113 |

Tabelle 2: (Fortsetzung)

| Bsp. Nr. | $R^1$ | $R^2$ | $R^3$ | $R^4$ | n | Physik. Daten (Schmelzpunkt (°C) |
|---|---|---|---|---|---|---|
| 56 | H | 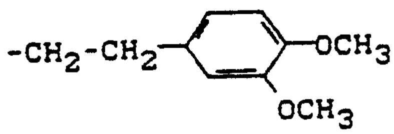 | Cl | - | 0 | 146 |
| 57 | H | 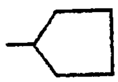 | Cl | - | 0 | 107 |
| 58 | 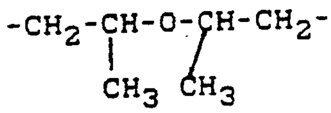 | | Cl | - | 0 | 159 |
| 59 | H | 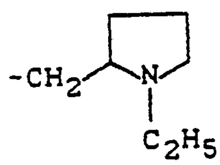 | Cl | - | 0 | 120 |
| 60 | H | 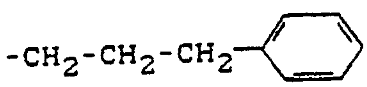 | Cl | - | 0 | 99 |
| 61 | H | $-CH_2-COOC_2H_5$ | Cl | - | 0 | 160 |
| 62 | H | 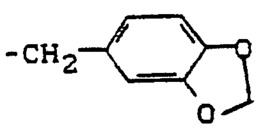 | $CF_3$ | (5-)$CF_3$ | 1 | 119 |

T a b e l l e 2: (Fortsetzung)

| Bsp. Nr. | $R^1$ | $R^2$ | $R^3$ | $R^4$ | n | Physik. Daten (Schmelzpunkt (°C) |
|---|---|---|---|---|---|---|
| 63 | H | 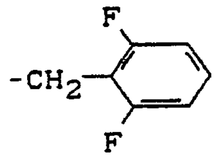 | Cl | - | 0 | 169 |
| 64 | H | 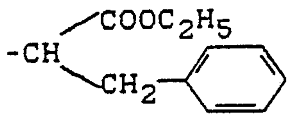 | Cl | - | 0 | 88 |
| 65 | H | 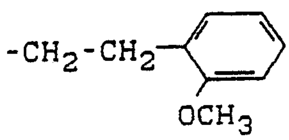 | Cl | - | 0 | 119 |
| 66 | H | $-CH_2-CN$ | Cl | - | 0 | 204 |
| 67 | H | $-CH_2-CH_2-CN$ | Cl | - | 0 | 176 |
| 68 | H | 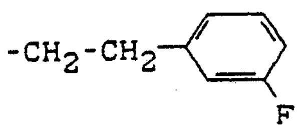 | $CF_3$ | - | 0 | 135 |
| 69 | H | $-CH_2-CH(OC_2H_5)_2$ | Cl | - | 0 | 107 |
| 70 | H | $-CH(CH(CH_3)_2)-COOC_2H_5$ | Cl | - | 0 | (Harz) |
| 71 | H | $-CH(CH_2CH(CH_3)_2)-COOCH_3$ | Cl | - | 0 | (Harz) |

T a b e l l e 2: (Fortsetzung)

| Bsp. Nr. | $R^1$ | $R^2$ | $R^3$ | $R^4$ | n | Physik. Daten (Schmelzpunkt (°C) |
|---|---|---|---|---|---|---|
| 72 | H | $-CH_2-$ 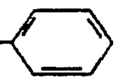 | $CF_3$ | (5-)Cl | 1 | 156 |
| 73 | H | $-CH_2-$ 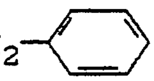 | Cl | (5-)$CF_3$ | 1 | 153 |
| 74 | H | $-CH_2-CO-NHCH_3$ | Cl | - | 0 | 220 |
| 75 | H | $-CH_2-$ 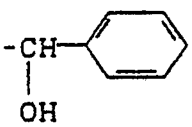 | $CF_3$ | - | 0 | 162 |
| 76 | H | $-CH_2-CH_2-$ 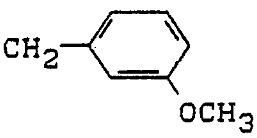 | $CF_3$ | - | 0 | 132 |
| 77 | H | $-CH_2-CN$ | $CF_3$ | (5-)$CF_3$ | 1 | 201 |
| 78 | H | $-CH_2-CH_2-CH(CH_3)_2$ | Cl | - | 0 | 86 |
| 79 | H | H | $CF_3$ | (5-)Cl | 1 | 227 |
| 80 | H | $-CH_2-CH_2-$ 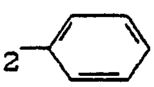 | $CF_3$ | (5-)Cl | 1 | 128 |
| 81 | H | $-CH_2-CH_2-CN$ | $CF_3$ | (5-)Cl | 1 | 187 |
| 82 | H | $-CH_2-CH_2-$ 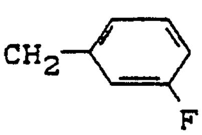 | $CF_3$ | (5-)Cl | 1 | 126 |

Tabelle 2: (Fortsetzung)

| Bsp. Nr. | $R^1$ | $R^2$ | $R^3$ | $R^4$ | n | Physik. Daten (Schmelzpunkt (°C) |
|---|---|---|---|---|---|---|
| 83 | H | $-CH_2-CH_2-$ 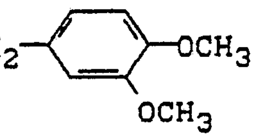 | $CF_3$ | (5-)Cl | 1 | 127 |
| 84 | H | $-CH-COOCH_3$<br>$CH(CH_3)_2$ | $CF_3$ | (5-)Cl | 1 | 107 |
| 85 | H | $-CH_2-CH_2-$ 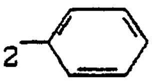 | Cl | (6-)Cl | 1 | 120 |
| 86 | H | $-CH_2-C(CH_3)_3$ | Cl | (6-)Cl | 1 | 164 |

Ausgangsstoffe der Formel (II)

Beispiel II-1:

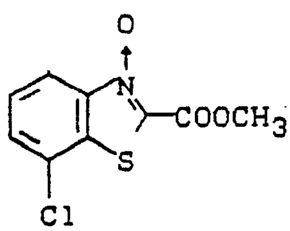

66 g (0,65 Mol) Triethylamin werden unter Rühren zu einer Mischung aus 115 g (0,60 Mol) 2,3-Dichlor-nitro-benzol, 63,6 g (0,60 Mol) Mercaptoessigsäure-methylester und 250 ml Dimethylsulfoxid tropfenweise gegeben. Durch äußere Kühlung wird dabei die Innentemperatur bei 40°C bis 50°C gehalten. Nach beendeter Aminzugabe wird das Reaktionsgemisch noch 12 Stunden bei 20°C gerührt. Dann wird mit Methanol verdünnt und das kristallin angefallene Produkt durch Absaugen isoliert.

Man erhält 108 g (73 % der Theorie) 7-Chlor-2-methoxy-carbonyl-benzthiazol-3-oxid vom Schmelzpunkt 159°C.

Analog Beispiel (II-1) können die in der nachstehenden Tabelle 3 aufgeführten Verbindungen der Formel (II) hergestellt werden.

Tabelle 3: Herstellungsbeispiele für die Verbindungen der Formel (II)

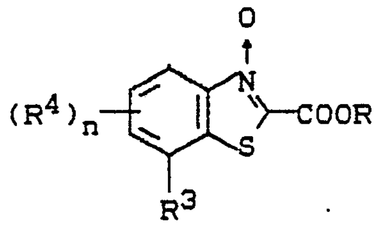

(II)

| Bsp. Nr. | R | $R^3$ | $R^4$ | n | Physik. Daten (Schmelzpunkt °C) |
|---|---|---|---|---|---|
| II-2 | $C_2H_5$ | Cl | - | 0 | 124 |
| II-3 | $C_4H_9$-n | Cl | - | 0 | 59 |
| II-4 | $CH_3$ | $CF_3$ | - | 0 | 128 |
| II-5 | $C_2H_5$ | $CF_3$ | (5-$CF_3$) | 1 | 62 |

## Verwendungsbeispiele

### Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25° C aufgestellt.

4 Tage nach der Inokulation erfolgt die Auswertung des Krankheitsbefalls.

Einen Wirkungsgrad von 80 - 90 % bei einer Wirkstoffkonzentration von 0,025 % zeigen z. B. die Verbindungen gemäß den Herstellungsbeispielen (1), (5), (9), (16), (19), (20), (22), (23), (25) und (28).

## Ansprüche

1. 7-substituierte 2-Carbonamido-benzthiazol-3-oxide der allgemeinen Formel (I)

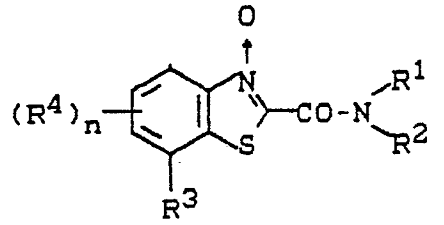

(I)

in welcher
n für die Zahlen 0, 1, 2 oder 3 steht,
$R^1$ für Wasserstoff oder gegebenenfalls substituiertes Alkyl steht,
$R^2$ für Wasserstoff, Hydroxy, Ureido oder für jeweils gegebenenfalls substituiertes Alkyl, Cycloalkyl, Cycloalkylalkyl, Alkenyl, Alkinyl, Alkoxy, Alkenyloxy, Aralkoxy, Aralkyl, Aryl, einen Heterocyclyl oder Heterocyclylalkyl steht,
oder
$R^1$ und $R^2$ zusammen für eine gegebenenfalls substituierte und gegebenenfalls durch Heteroatome unterbrochene Alkylenkette stehen,
$R^3$ für Halogen, Alkyl oder Halogenalkyl steht und $R^4$ für Halogen, Alkyl oder Halogenalkyl steht.

2. Substituierte 2-Carbonamido-benzthiazol-3-oxide gemäß Anspruch 1, wobei in der Formel (I)
n für die Zahlen 0, 1 oder 2 steht,
$R^1$ für Wasserstoff oder für geradkettiges oder verzweigtes Alkyl mit 1 bis 6 Kohlenstoffatomen steht, welches gegebenenfalls ein- bis dreifach, gleich oder verschieden durch Halogen, Hydroxy, $C_1$-$C_4$-Alkoxy, $C_1$-$C_4$-Alkoxy-carbonyl und/oder Di-($C_1$-$C_4$-alkyl)-amino substituiert ist,
$R^2$ für Wasserstoff, Hydroxy, Ureido, für geradkettiges oder verzweigtes Alkyl mit 1 bis 6 Kohlenstoffatomen steht, welches gegebenenfalls ein- bis dreifach, gleich oder verschieden durch Halogen, Hydroxy, Cyano, Benzoyl, $C_1$-$C_4$-Alkoxy, $C_1$-$C_4$-Alkoxy-carbonyl, $C_1$-$C_4$-Alkylamino-carbonyl und/oder Di-($C_1$-$C_4$-alkyl)-amino substituiert ist, für Cycloalkyl mit 3 bis 6 Kohlenstoffatomen oder Cycloalkylalkyl mit 3 bis 6 Kohlenstoffatomen im Cycloalkylteil und 1 bis 3 Kohlenstoffatomen im Alkylteil steht, welche gegebenenfalls ein- bis dreifach, gleich oder verschieden durch Halogen und/oder $C_1$-$C_4$-Alkyl substituiert sind, für geradkettiges oder verzweigtes Alkenyl mit 3 bis 6 Kohlenstoffatomen steht, welches gegebenenfalls ein- bis dreifach, gleich oder verschieden durch Halogen, Cyano und/oder $C_1$-$C_4$-Alkoxy-carbonyl substituiert ist, für geradkettiges oder verzweigtes Alkinyl mit 3 bis 6 Kohlenstoffatomen steht, welches gegebenenfalls ein- bis dreifach, gleich oder verschieden durch Halogen, Cyano und/oder $C_1$-$C_4$-Alkoxy-carbonyl substituiert ist, für geradkettiges oder verzweigtes Alkoxy mit 1 bis 6 Kohlenstoffatomen steht, welches gegebenenfalls ein- bis dreifach, gleich oder verschieden durch Halogen, Cyano, $C_1$- $C_4$-Alkoxy und/oder $C_1$-$C_4$-Alkoxy-carbonyl substituiert ist, für geradkettiges oder verzweigtes Alkenyloxy mit 3 bis 6 Kohlenstoffatomen steht, welches gegebenenfalls ein- bis dreifach, gleich oder verschieden durch Halogen substituiert ist, für Benzyloxy steht, welches gegebenenfalls ein- bis dreifach, gleich oder verschieden durch Halogen, Nitro, Cyano, $C_1$-$C_4$-Alkyl, $C_1$-$C_4$-Alkoxy, $C_1$-$C_4$-Alkoxy-carbonyl und/oder Halogenalkyl mit 1 oder 2 Kohlenstoffatomen und 1 bis 5 gleichen oder verschiedenen Halogenatomen substituiert ist, für Aralkyl mit 6 bis 10 Kohlenstoffatomen im Arylteil und 1 bis 4 Kohlenstoffatomen im geradkettigen oder verzweigten Alkylteil steht, welches gegebenenfalls im Aryl- und/oder Alkylteil jeweils ein- bis dreifach, gleich oder verschieden durch Halogen, Hydroxy, Phenyl, Nitro, Cyano, $C_1$-$C_4$-Alkyl, $C_1$-$C_4$-Alkoxy, $C_1$-$C_4$-Alkoxy-carbonyl und/oder Halogenalkyl mit 1 oder 2 Kohlenstoffatomen und 1 bis 5 gleichen oder verschiedenen Halogenatomen substituiert ist, für Phenyl oder Naphthyl steht, welche gegebenenfalls ein- bis dreifach, gleich oder verschieden durch Halogen, Hydroxy, Nitro, Cyano, $C_1$-$C_4$-Alkyl, Halogenalkyl mit 1 oder 2 Kohlenstoffatomen und 1 bis 5 gleichen oder verschiedenen Halogenatomen, $C_1$-$C_4$-Alkoxy, $C_1$-$C_2$-Halogenalkoxy, $C_1$-$C_4$-Alkylthio, $C_1$-$C_2$-Halogenalkylthio und/oder $C_1$-$C_4$-Alkoxy-carbonyl substituiert sind, oder für einen gegebenenfalls durch Halogen, Nitro, Cyano, $C_1$-$C_4$-Alkyl, $C_1$-$C_4$-Alkoxy und/oder $C_1$-$C_4$-Alkoxy-carbonyl ein- bis dreifach, gleich oder verschieden substituierte Ein-oder Zweiring-Heterocyclus steht, wie Furyl, Thienyl, Oxazolyl, Thiazolyl, Oxadiazoly, Thiadiazolyl, Pyridyl, Pyrimidinyl, Pyrrolidinyl, Thiomorpholinyl, Morpholinyl

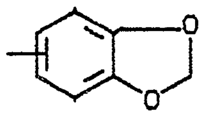

oder

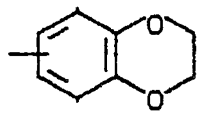

steht, welche gegebenenfalls über eine geradkettige oder verzweigte Alkylenkette mit 1 bis 4 Kohlenstoffatomen gebunden sind

oder

$R^1$ und $R^2$ zusammen für eine gegebenenfalls ein- bis dreifach, gleich oder verschieden durch $C_1$-$C_4$-Alkyl substituierte und gegebenenfalls durch ein oder mehrere Sauerstoffatome unterbrochene Alkylenkette mit 2 bis 8 Ringgliedern stehen,

$R^3$ für Halogen, Methyl, Ethyl oder Halogenalkyl mit 1 bis 3 Kohlenstoffatomen und 1 bis 6 gleichen oder verschiedenen Halogenatomen steht und

$R^4$ für Halogen, Methyl, Ethyl oder für Halogenalkyl mit 1 bis 3 Kohlenstoffatomen und 1 bis 6 gleichen oder verschiedenen Halogenatomen steht.

3. 7-Substituierte 2-Carbonamido-benzthiazol-3-oxide gemäß Anspruch 1, wobei in der Formel (I)

n für die Zahlen 0, 1 oder 2 steht,

$R^1$ für Wasserstoff oder für geradkettiges oder verzweigtes Alkyl mit 1 bis 4 Kohlenstoffatomen steht, welches gegebenenfalls ein- oder zweifach, gleich oder verschieden durch Hydroxy, $C_1$-$C_2$-Alkoxy oder $C_1$-$C_2$-Alkoxy-carbonyl substituiert ist,

$R^2$ für Wasserstoff, Hydroxy, Ureido, für geradkettiges oder verzweigtes Alkyl mit 1 bis 6 Kohlenstoffatomen steht, welches gegebenenfalls ein- oder zweifach, gleich oder verschieden durch Hydroxy, Cyano, Benzoyl, $C_1$-$C_2$-Alkoxy, $C_1$-$C_2$-Alkoxy-carbonyl, $C_1$-$C_2$-Alkylamino-carbonyl oder Di-($C_1$-$C_2$-alkyl)-amino substituiert ist, für Cycloalkyl mit 3 bis 6 Kohlenstoffatomen, für Cycloalkylmethyl oder -ethyl mit 3 bis 6 Kohlenstoffatomen im Cycloalkylteil, für geradkettiges oder verzweigtes Alkenyl mit 3 bis 6 Kohlenstoffatomen, für geradkettiges oder verzweigtes Alkinyl mit 3 bis 6 Kohlenstoffatomen, für geradkettiges oder verzweigtes Alkoxy mit 1 bis 6 Kohlenstoffatomen, für geradkettiges oder verzweigtes Alkenyloxy mit 3 bis 6 Kohlenstoffatomen oder für Benzyloxy steht, welches gegebenenfalls ein- oder zweifach, gleich oder verschieden durch Fluor, Chlor, Methyl und/oder Methoxy substituiert ist, für Phenylalkyl mit 1 bis 3 Kohlenstoffatomen im Alkylteil steht, welches gegebenenfalls ein- bis dreifach, gleich oder verschieden im Phenyl- und/oder Alkylteil durch Fluor, Chlor, Hydroxy, Phenyl, $C_1$-$C_4$-Alkyl, $C_1$-$C_2$-Alkoxy, Trifluormethyl und/oder $C_1$-$C_2$-Alkoxy-carbonyl substituiert ist, für Naphthylmethyl oder für Phenyl steht, welches gegebenenfalls ein- bis dreifach, gleich oder verschieden durch Fluor, Chlor, Brom, Hydroxy, $C_1$-$C_4$-Alkyl, Trifluormethyl, $C_1$-$C_2$-Alkoxy, Trifluormethoxy, $C_1$-$C_2$-Alkylthio und/oder Trifluormethylthio substituiert ist, für Furylmethyl, für Pyridylmethyl, für gegebenenfalls ein- oder zweifach durch Methyl substituiertes Morpholinyl, für Morpholinyl-$C_1$-$C_3$-alkyl oder für gegebenenfalls ein- oder zweifach, gleich oder verschieden durch $C_1$-$C_3$-Alkyl substituiertes Pyrrolidinyl-$C_1$-$C_3$-alkyl steht,

für

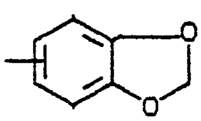

,

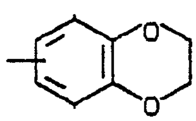

oder

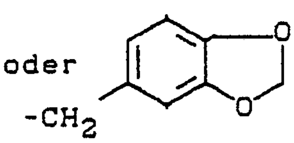

steht,

oder

$R^1$ und $R^2$ zusammen mit dem Stickstoffatom, an das sie gebunden sind, für gegebenenfalls ein- bis dreifach, gleich oder verschieden durch Methyl und/ oder Ethyl substituiertes Pyrrolidinyl, Piperidinyl oder Morpholinyl stehen,

$R^3$ für Chlor oder Trifluormethyl steht und

$R^4$ für Chlor oder Trifluormethyl steht.

4. Verfahren zur Herstellung von 7-substituierten 2-Carbonamido-benzthiazol-3-oxiden der allgemeinen Formel (I)

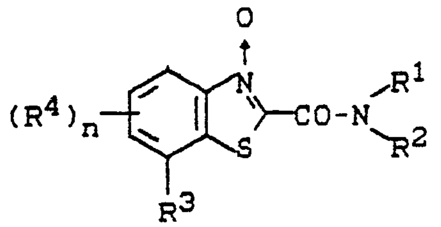

(I)

in welcher
n für die Zahlen 0, 1, 2 oder 3 steht,
$R^1$ für Wasserstoff oder gegebenenfalls substituiertes Alkyl steht,
$R^2$ für Wasserstoff, Hydroxy, Ureido oder für jeweils gegebenenfalls substituiertes Alkyl, Cycloalkyl, Cycloalkylalkyl, Alkenyl, Alkinyl, Alkoxy, Alkenyloxy, Aralkoxy, Aralkyl, Aryl, Heterocyclyl oder Heterocyclylalkyl steht,
oder
$R^1$ und $R^2$ zusammen für eine gegebenenfalls substituierte und gegebenenfalls durch Heteroatome unterbrochene Alkylenkette stehen,
$R^3$ für Halogen, Alkyl oder Halogenalkyl steht und
$R^4$ für Halogen, Alkyl oder Halogenalkyl steht,
dadurch gekennzeichnet, daß man 7-substituierte 2-Alkoxycarbonyl-benzthiazol-3-oxide der allgemeinen Formel (II)

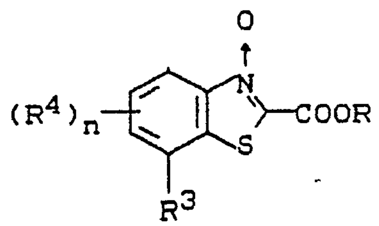

(II)

in welcher
n, $R^3$ und $R^4$ die oben angegebenen Bedeutungen haben und
R für Alkyl steht,
mit Aminen der allgemeinen Formel (III)

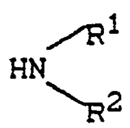

(III)

in welcher
$R^1$ und $R^2$ die oben angegebenen Bedeutungen haben, gegebenenfalls in Gegenwart von Verdünnungsmitteln umsetzt.

5. Schädlingsbekämpfungsmittel, gekennzeichnet durch einen Gehalt an mindestens einem 7-substituierten 2-Carbonamido-benzthiazol-3-oxid der Formel (I) nach den Ansprüchen 1 oder 4.

6. Verwendung von 7-substituierten 2-Carbonamido-benzthiazol-3-oxiden der Formel (I) nach den Ansprüchen 1 oder 4 zur Bekämpfung von Schädlingen.

7. Verfahren zur Bekämpfung von Schädlingen, dadurch gekennzeichnet, daß man 7-substituiertes 2-Carbonamido-benzthiazol-3-oxid der Formel (I) nach den Ansprüchen 1 oder 4 auf Schädlinge und/oder ihren Lebensraum einwirken läßt.

8. Verfahren zur Herstellung von Schädlingsbekämpfungsmitteln, dadurch gekennzeichnet, daß man 7-substituierte 2-Carbonamido-benzthiazol-3-oxide der Formel (I) nach den Ansprüchen 1 oder 4 mit Streckmitteln und/oder oberflächenaktiven Mitteln vermischt.

9. 7-Substituierte 2-Alkoxycarbonyl-benzthiazol-3-oxide der allgemeinen Formel (II)

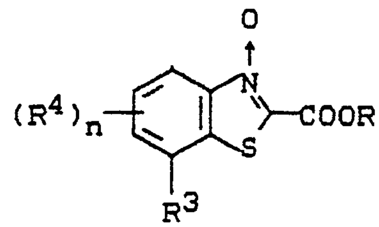

(II)

in welcher
n für die Zahlen 0, 1, 2 oder 3 steht,
$R^3$ für Halogen, Alkyl oder Halogenalkyl steht,
$R^4$ für Halogen, Alkyl oder Halogenalkyl steht und
R für Alkyl steht.

10. Verfahren zur Herstellung von 7-substituierten 2-Alkoxycarbonyl-benzthiazol-3-oxiden der Formel (II)

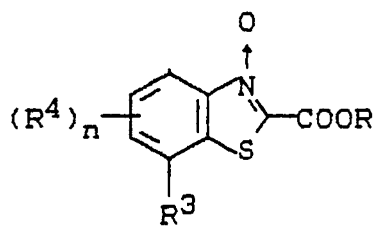

(II)

in welcher
n für die Zahlen 0, 1, 2 oder 3 steht,
$R^3$ für Halogen, Alkyl oder Halogenalkyl steht,
$R^4$ für Halogen, Alkyl oder Halogenalkyl steht und
R für Alkyl steht,
dadurch gekennzeichnet, daß man
2-Chlor-nitrobenzol-Derivate der allgemeinen Formel (IV)

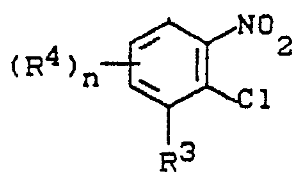

(IV)

in welcher
n, $R^3$ und $R^4$ die oben angegebenen Bedeutungen haben,
mit Mercaptoessigsäureestern der allgemeinen Formel (V)
$HS-CH_2-COOR$ (V)
in welcher
R die oben angegebene Bedeutung hat,
in Gegenwart einer Base und in Gegenwart eines Verdünnungsmittels bei Temperaturen zwischen 0 °C und 100 °C umsetzt.

Europäisches Patentamt

# EUROPÄISCHER RECHERCHENBERICHT

Nummer der Anmeldung: EP 89104342.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 136 924 (FARBENFABRIKEN BAYER AG)<br>* Anspruch 1; Beispiele 1,3,13; Seite 5, letzter Absatz * | 1,4-8, 9,10 | C 07 D 277/68<br>C 07 D 417/12<br>A 01 N 43/78 |
| A | DE - A1 - 2 136 923 (BAYER AG)<br>* Ansprüche; Beispiele 6,13, 22,24; Seite 5, letzter und vorletzter Absatz * | 1,4-8, 9,10 | |
| P,A | CHEMICAL ABSTRACTS, Band 109, Nr. 19, 7. November 1988, Columbus, Ohio, USA<br>CHI, CH. et al. "Studies on Anorine--containing heterocyclic compounds. 3. Reactions of 3,5-dinitro-2--chlorobenzotrifluoride and 3,5--dinitro-4-chlorobenzotrifluoride with sulfur and nitrogen-containing nucleophiles"<br>Seite 709, Spalte 1, Zusammenfassung-Nr. 170 291g<br>& J. Fluorine Chem. 1988, 38(3), 327-34 | 9,10 | RECHERCHIERTE SACHGEBIETE (Int. Cl.4)<br>C 07 D 277/00<br>C 07 D 417/00 |
| A | CHEMICAL ABSTRACTS, Band 80, Nr. 19, 13. Mai 1974, Columbus, Ohio, USA<br>WAGNER, K. et al. "Benzothiazole N-oxides. III. Synthesis of ethyl 1-(arylimino) ($S^{IV}$) benzothiazole - 2 - carboxylates"<br>Seite 410, Spalte 2, Zusammenfassung-Nr. 108 420c<br>& Chem. Ber. 1974, 107(2), 414-23 | 9,10 | |

Der vorliegende Recherchenbericht wurde für alle Patentansprüche erstellt.

| Recherchenort | Abschlußdatum der Recherche | Prüfer |
|---|---|---|
| WIEN | 23-05-1989 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

# EUROPÄISCHER RECHERCHENBERICHT

Nummer der Anmeldung



EP 89104342.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 80, Nr. 19, 13. Mai 1974, Columbus, Ohio, USA<br>WAGNER, K. et al. "Benzothiazole N-oxides. II. Synthesis of substituted 2(3H)-benzothiazolones" Seite 411, Spalte 1, Zusammenfassung-Nr. 108 422e<br>& Chem. Ber. 1974, 107(1), 305-16<br>---- | 1,4,9, 10 | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| WIEN | 23-05-1989 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument